**Europäisches Patentamt**

**European Patent Office**

**Office européen des brevets**

(11) Publication number: **0 086 062**
**B1**

(12) **EUROPEAN PATENT SPECIFICATION**

(45) Date of publication of patent specification: **19.04.89**

(51) Int. Cl.⁴: **A 61 K 6/10**

(21) Application number: **83300420.3**

(22) Date of filing: **27.01.83**

(54) **Method for regulating the working time of materials for taking impressions.**

(30) Priority: **05.02.82 IT 932282**
**05.02.82 IT 932382**

(43) Date of publication of application:
**17.08.83 Bulletin 83/33**

(45) Publication of the grant of the patent:
**19.04.89 Bulletin 89/16**

(84) Designated Contracting States:
**AT BE CH DE FR GB LI NL SE**

(56) References cited:
**FR-A- 978 025**

**"Guide to dental materials and devices", 7th edition, 1974-1975, pages 71-73,79, American Dental Assocation, Chicago, USA;**

(73) Proprietor: **Cozzi, Gualtiero**
**28 Via Domenico Buonvicini**
**I-50132 Firenze (IT)**

(72) Inventor: **Cozzi, Gualtiero**
**28 Via Domenico Buonvicini**
**I-50132 Firenze (IT)**

(74) Representative: **Corin, Christopher John et al Mathisen Macara & Co. The Coach House 6-8 Swakeleys Road**
**Ickenham Uxbridge UB10 8BZ (GB)**

Courier Press, Leamington Spa, England.

## Description

The present invention relates to a method of preparing a material for taking an impression comprising mixing an alginate-based material with water to produce a viscous mass.

The invention particularly relates to materials for taking dental impressions, in particular to those based on alginates, and commonly known under the name of "Alginates", which have the following main components, which are in the fine powder state:

a) a water-soluble alginate (for example sodium or potassium alginate);

b) a slightly water-soluble calcium salt (for example sulphate, tartrate, citrate);

c) an inert material (such as diatomaceous earth);

d) a setting retarder (for example phosphates of alkaline metals in their various forms, EDTA salts).

A mixture, in suitable proportions, of the components (a), (b), (c), (d) with water provides a viscous mass which can be moulded, stretched and generally kneaded for a time which is longer the greater the percentage of the retarder (d). The mix then sets and provides a mass characterised by high elasticity qualities. This viscous mass is the material used in the dental profession for obtaining impressions of the oral cavity, of the denture or of a part thereof, and for obtaining impressions for duplicating patterns. The elasticity of the product after setting makes it easy to detach the impression from the denture or from the pattern without breakage or substantial deformation, and enables undercuts to be incorporated.

All the aforesaid impression materials are characterised by their mixing time, their working time, and their setting time.

The mixing time indicates the time necessary for making the mixture of powder and water homogeneous by working it with a spatula. For reasons of practical application this must not exceed one minute, but cannot be reduced to too short a time in order not to compromise the homogeneousness of the mixture, and consequently in order not to compromise the perfection of the impression.

The working time comprises both the mixing time and the time which the operator has available for filling the impression-carrying scoop and for carrying out all the operations which precede the application of the material to the patient's mouth.

The setting time indicates the period between the commencement of mixing and the completion of setting. In other words, the setting time corresponds to the sum of the working time and the time for which it remains in the patient's mouth.

A knowledge of the "working time" is of particular importance for the correct use of impression materials. If the working time is longer than that scheduled, the patient suffers considerable discomfort, being thus compelled to support a more or less running viscous mass in the mouth. Vomit-inducing stimuli in such cases are not infrequent. If the working time is shorter than scheduled, the materials sets before use (it sets in the hand) and must be thrown away.

Unfortunately, the working time cannot be evaluated previously. Not even the manufacturer is able to supply precise data, because this depends largely on the ambient temperature at the time of use, and the hardness of the water used for mixing. In this respect, this time becomes shorter as the temperature and/or hardness of the water increase, and in contrast it increases substantially as the temperature reduces and/or water low in calcium salts is used.

Exact knowledge of the mixing time and working time has been attained by introducing special "chromatic phase indicators" into the impression material These indicators are constituted by colouring substances which change colour in relation to the attainment of a particular stage in the reaction process preceding setting, and they thus provide an extemporaneous indication of the effective mixing time and working time whatever the temperature of the powder and water, and whatever the hardness of the water, the evaluation being available at any moment during the various operations.

The introduction — when using "Alginates" — of chromatic phase indicators has brilliantly solved the problem of knowing the mixing and working times. However, if these are too long or too short, it is not possible to act in order to vary them. This has caused many manufacturers — whether they use chromatic indication or not — to make the same product available to operators in two different types, namely rapid setting and normal setting. The former is more suitable for the winter period or in relatively cold environmental situations, while the latter is more suitable for the summer period or in distinctly warm environmental situations. This solution to the problem is only partial, and itself leads to other non-negligible drawbacks in terms of production, distribution and use.

The problem could be held to be satisfactorily solved only if the operator is provided with the possibility of regulating at will the working time of the alginate in relation to the environment in which he works, and in relation to the water which he uses. This is attainable by the method according to the invention.

The method makes it possible to regulate at will the mixing time and working time of materials for taking dental impressions, or alternatively serves for neutralising the accelerating effect which an increase in the working environment temperature and/or the hardness of the water used for mixing have on the mixing and working time of alginate-based materials.

The method of the present invention is characterised by adding at the mixing stage a lithium salt setting retarder in an amount selected to produce a desired working time in dependence on the working conditions and intrinsic properties of the alginate-based material and water.

During the daily work of the professional practitioner, the required regulation of the working time can be carried out at the moment of preparation of the mixture of water and alginate, by adding in controlled quantities small suitable amounts of the retarder in the solid state to a rapid-setting alginate in powder form, or small quantities of soluble retarder, either in the liquid phase or in solution, to the water.

The lithium salt can be made avaiable in the solid state for adding to the impression material power or in a soluble solid state or in solution for adding to the water used for mixing, in quantities controllable at will.

The retarder — in powder, tablet, solution or other form — can be provided directly by the manufacturer, either together with or without the alginate pack.

As stated heretofore, the retarders are suitably chosen by the alginate manufacturer for addition in controlled quantities to the powder or water at the moment of use.

Advantageously, the lithium salt setting retarder is a chloride, nitrate, perchloride, sulphate or carbonate.

This method is particularly suitable for use with alginate-based impression materials comprising a tribasic alkaline orthophosphate retarder or the like.

It has been shown experimentally that lithium salts, when introduced in small quantities into alginates of which the retarder consists mainly of tribasic alkaline orthophosphate, increase the effectiveness of said retarder for the entire period of its action. The lithium cation acts in an exclusively physical manner on the kinetics of the reactive processes which lead to setting, thus modifying the diffusion layer which controls the transfer of the calcium ions from the surface of the crystals, for example of calcium sulphate, to the solution.

The lithium salts do not alter the stoichiometric balance of the reactions which are concluded by setting. For this reason they have no negative action on the physical properties of the mass which forms the impression.

The lithium salts can thus be considered as specific controllers of the working time, in that they do not in any way affect the time of application or the resting time in the patient's mouth. This is a further positive effect of their use, given that the resting time in the mouth — being practically uninfluenced by the temperature of the environment in which mixing is carried out — requires no regulation, and in fact should be as short as possible.

The required regulation of the working time during the professional practitioner's daily activity can be carried out at the moment of preparing the mixture of water and alginate, by adding, in controlled quantity, small suitable amounts of a lithium salt in the solid state (any lithium salt can be used) to the rapid-setting "Alginate" in powder form, or small doses of the lithium salt in tablet form or in solution to the water.

## Claims

1. A method of preparing a material for taking an impression comprising mixing an alginate-based material with water to produce a viscous mass characterised by adding at the mixing stage a lithium salt setting retarder in an amount selected to produce a desired working time in dependence on the working conditions and intrinsic properties of the alginate-based material and water.

2. A method as claimed in claim 1, characterised in that the alginate-based material includes a setting retarder which is or is equivalent to a tribasic alkaline orthophosphate.

3. A method as claimed in claim 1 or 2, characterised in that the lithium salt is lithium chloride, lithium nitrate, lithium perchorate, lithium sulphate, or lithium carbonate.

4. A method as claimed in any one of the preceding claims, characterised in that the regulator is added in the solid state.

5. A method as claimed in any one of claims 1 to 3, characterised in that the regulator is added in a liquid state and is provided either as a liquid or as a solid for the preparation of a solution.

6. A method as claimed in any one of the preceding claims for preparing a material for taking a dental impression.

## Patentansprüche

1. Verfahren zur Zubereitung eines Abdruckmaterials, bei dem ein Material auf Alginatbasis mit Wasser gemischt wird, un eine viskose Masse zu erhalten, dadurch gekennzeichnet, daß in der Mischstufe ein Lithiumsalz als Aushärteverzögerer zugesetzt wird, dessen Anteil so gewählt wird, daß eine gewünschte Verarbeitungszeit in Abhängigkeit von den Arbeitsbedingungen und den spezifischen Eigenschaften des Materials auf Alginatbasis und des Wassers erzielt wird.

2. Verfahren nach Anspruch 1, dadurch gekennzeichnet, daß das Material auf Alginatbasis einen Aushärteverzögerer enthält, der ein tribasischalkalisches Orthophosphat oder ein Äquivalent davon ist.

3. Verfahren nach Anspruch 1 oder 2, dadurch gekennzeichnet, daß das Lithiumsalz Lithiumchlorid, Lithiumnitrat, Lithiumperchlorat, Lithiumsulfat oder Lithiumcarbonat ist.

4. Verfahren nach einem der vorhergehenden Ansprüche; dadurch gekennzeichnet, daß der Verzögerer in festem Zustand zugesetzt wird.

5. Verfahren nach einem der Ansprüche 1 bis 3, dadurch gekennzeichnet, daß der Verzögerer in flüssigem Zustand zugesetzt wird und entweder aus einer Flüssigkeit oder einem für die Herstellung einer Lösung geeigneten festen Stoff besteht.

6. Verfahren nach einem der vorhergehenden Ansprüche zum Zubereiten eines Materials zum Abnehmen dentaler Abdrücke.

## Revendications

1. Procédé pour préparer une matière pour la prise d'une empreinte comprenant le mélange d'une matière a base d'alginate avec de l'eau pour produire une masse visqueuse, caractérisé par l'addition, à l'étape de mélange, d'un retardateur de prise à base de sel de lithium en une quantité choisie pour produire un temps de travail désiré, dépendant des conditions de travail et des propriétés intrinsèques de la matière à base d'alginate et d'eau.

2. Procédé selon la revendication 1, caractérisé en ce que la matière à base d'alginate comprend un retardateur de prise qui est un orthophoshate trialcalin ou est équivalent à un orthophosphate trialcalin.

3. Procédé selon la revendication 1 ou 2, caractérisé en ce que le sel de lithium est le chlorure de lithium, le nitrate de lithium, le perchlorate de lithium, le sulfate de lithium ou le carbonate de lithium.

4. Procédé selon l'une quelconque des revendications précédentes, caractérisé en ce que le régulateur est ajoute à l'état solide.

5. Procédé selon l'une quelconque des revendications 1 à 3, caractérisé en ce que le régulateur est ajouté dans un étant liquide et est fourni soit sous formé d'un solide, soit sous forme d'un liquide pour la préparation d'une solution.

6. Procédé selon l'une quelconque des revendications précédentes pour la préparation d'une matière pour prendre une empreinte dentaire.